# EUROPEAN PATENT SPECIFICATION

(11) **EP 4 637 674 B1**
(45) Date of publication and mention of the grant of the patent: **11.11.2026**
(21) Application number: 23825519.4
(22) Date of filing: 28.11.2023
(51) Int. Cl.: A61K 8/02, A61K 8/55, A61K 8/46, A61K 8/73, A61Q 19/00

(54) **COMPOSITION COMPRISING POLYION COMPLEX WITH LOW MOLECULAR WEIGHT CHITOSANS**
ZUSAMMENSETZUNGEN AUS EINEM POLYIONISCHEN KOMPLEX UND EINEM NIEDERMOLEKULAREN CHITOSAN.
COMPOSITIONS COMPRENANT UN COMPLEXE POLYIONIQUE ET UN CHITOSAN DE FAIBLE POIDS MOLÉCULAIRE.

(30) Priority: 19.12.2022 JP 2022202285; 27.01.2023 FR 2300773
(43) Date of publication of application: 29.10.2025
(73) Proprietor: L'OREAL, 75008 Paris (FR); Yamamoto, Mariko, Kawasaki-shi, Kanagawa, 213-0012 (JP); Kasai, Takehiko, Kanagawa 2130012 (JP); Shiroya, Toshifumi, Kawasaki-shi, Kanagawa 2130012 (JP); Okuda, Shinsuke, Kawasaki-shi, Kanagawa 2130012 (JP); Isojima, Tatsushi, Kawasaki-shi, Kanagawa, 213-0012 (JP)
(72) Inventor: YAMAMOTO Mariko, Kawasaki-shi, Kanagawa 2130012 (JP); KASAI Takehiko, Kawasaki-shi, Kanagawa 2130012 (JP); SHIROYA Toshifumi, Kawasaki-shi, Kanagawa 2130012 (JP); OKUDA Shinsuke, Kawasaki-shi, Kanagawa 2130012 (JP); ISOJIMA Tatsushi, Kawasaki-shi, Kanagawa 2130012 (JP)
(74) Representative: Ipsilon
(86) International application number: PCT/JP2023/043206
(87) International publication number: WO 2024/135302

(56) References cited:
- WO-A1-2017/104221
- WO-A1-2018/230673
- WO-A1-2018/230739
- WO-A1-2018/230740
- WO-A1-2020/045134
- WO-A1-2021/125069
- WO-A1-2021/171909
- FR-A1- 3 118 700

## Description

### TECHNICAL FIELD

The present invention relates to a composition including polyion complex, as well as a cosmetic process using the composition.

### BACKGROUND ART

A polyion complex, which may be in the form of a particle and can be formed with a cationic polymer and an anionic polymer, has already been known.

For Example, WO 2021/125069 discloses a composition which is useful for cosmetic treatments and comprises, in one embodiment, at least one polyion complex particle comprising at least one cationic polymer, at least one hyaluronic acid-based anionic polymer and at least one non-polymeric acid having two or more pKa values or a salt thereof. In the prior art disclosed in WO 2021/125069 uses poly-ε-lysine as the cationic polymer.

WO 2018/230673 further discloses a cosmetic composition, comprising: (a) at least one particle, comprising at least one cationic polymer and at least one amphoteric polymer, at least one anionic polymer and at least one amphoteric polymer, or at least one amphoteric polymer, and at least one non-polymeric acid having two or more pKa values or salt(s) thereof .WO 2018/230739 discloses a cosmetic composition comprising: (a) at least one particle, comprising at least one cationic polymer, at least one anionic polymer, and at least one non-polymeric base having two or more pKb values or salt(s) thereof.

### DISCLOSURE OF INVENTION

There still is a need for a stable composition which can form a polyion complex and provide good spreadability during application and improved texture such as reduced stickiness after application.

Thus, an objective of the present invention is to provide a stable composition which is capable of forming a polyion complex and providing good spreadability during application and improved texture such as reduced stickiness after application.

The above objective of the present invention can be achieved by a composition, comprising:
(a) at least one cationic polymer:
(b) at least one anionic polymer;
(c) at least one non-polymeric acid having two or more pKa values or salt(s) thereof; and
(d) water,
wherein
the (a) cationic polymer is selected from chitosans with a molecular weight of 20,000 or less.

The amount of the (a) cationic polymer(s) in the composition according to the present invention may be from 0.01% to 15% by weight, preferably from 0.05% to 10% by weight, and more preferably from 0.1% to 5% by weight, relative to the total weight of the composition.

The (b) anionic polymer may have at least one negatively chargeable and/or negatively charged moiety selected from the group consisting of a sulfuric group, a sulfate group, a sulfonic group, a sulfonate group, a phosphoric group, a phosphate group, a phosphonic group, a phosphonate group, a carboxylic group, and a carboxylate group, and preferably selected from the group consisting of a carboxylic group and a carboxylate group.

The (b) anionic polymer may be selected from the group consisting of polysaccharides such as alginic acid, hyaluronic acid, and cellulose polymers, anionic (co)polyaminoacids such as (co)polyglutamic acids, (co)poly(meth)acrylic acids, (co)polyamic acids, (co)polystyrene sulfonate, (co)poly(vinyl sulfates), dextran sulfate, chondroitin sulfate, (co)polymaleic acids, polyfumaric acids, maleic acid (co)polymers, and salts thereof.

The (b) anionic polymer may be selected from polysaccharides, preferably hyaluronic acid and derivatives thereof, cellulose polymers and salts thereof, and a mixture thereof, and more preferably hyaluronic acid and salts thereof, carboxymethylcellulose and salts thereof, and a mixture thereof.

The amount of the (b) anionic polymer(s) in the composition according to the present invention may be from 0.01% to 15% by weight, preferably from 0.05% to 10% by weight, and more preferably from 0.1% to 5% by weight, relative to the total weight of the composition.

The (c) non-polymeric acid having two or more pKa values or salt(s) thereof may be an organic acid or salt(s) thereof, preferably a hydrophilic or water-soluble organic acid or salt(s) thereof, and more preferably phytic acid or salts thereof, terephthalylidene dicamphor sulfonic acid or salts thereof, or a mixture thereof.

The amount of the (c) non-polymeric acid having two or more pKa values or salt(s) thereof in the composition according to the present invention may be from 0.001% to 10% by weight, preferably from 0.005% to 5% by weight, and more preferably from 0.01% to 1% by weight, relative to the total weight of the composition.

The amount of the (d) water in the composition according to the present invention may be from 50% to 99% by weight, preferably from 60% to 98% by weight, and more preferably from 70% to 97% by weight, relative to the total weight of the composition.

The pH of the composition according to the present invention may be from 3 to 9, preferably from 3.5 to 8.5, and more preferably from 4 to 8.

The composition according to the present invention may be a cosmetic composition, preferably a skin cosmetic composition, and more preferably a skin care cosmetic composition.

The present invention also relates to a cosmetic process for a keratin substance such as skin, comprising
applying to the keratin substance the composition according to the present invention; and drying the composition to form a cosmetic film on the keratin substance.

The present invention may also relate to a use of (a) at least one cationic polymer, wherein the (a) cationic polymer is selected from chitosans with a molecular weight of 20,000 or less, in a composition comprising:
(b) at least one anionic polymer;
(c) at least one non-polymeric acid having two or more pKa values or salt(s) thereof; and
(d) water,

in order to stabilize the composition and/or improve the spreadability of the composition.

The present invention may also relate to a film, preferably a cosmetic film, comprising:
(a) at least one cationic polymer;
(b) at least one anionic polymer; and
(c) at least one non-polymeric acid having two or more pKa values or salt(s) thereof,
wherein
the (a) cationic polymer is selected from chitosans with a molecular weight of 20,000 or less.

The present invention may also relate to a use of (a) at least one cationic polymer, wherein the (a) cationic polymer is selected from chitosans with a molecular weight of 20,000 or less, in a film comprising:
(b) at least one anionic polymer; and
(c) at least one non-polymeric acid having two or more pKa values or salt(s) thereof,

in order to improve the texture of film, preferably in order to enhance moisturizing feeling, softness and smoothness provided by the film and/or to reduce stickiness of the film, and more preferably in order to reduce the stickiness of the film

### BEST MODE FOR CARRYING OUT THE INVENTION

After diligent research, the inventors have discovered that it is possible to provide a stable composition which is capable of forming a polyion complex and providing good spreadability during application and improved texture such as reduced stickiness after application.

Thus, the composition according to the present invention comprises:
(a) at least one cationic polymer:
(b) at least one anionic polymer;
(c) at least one non-polymeric acid having two or more pKa values or salt(s) thereof; and
(d) water,
wherein
the (a) cationic polymer is selected from chitosans with a molecular weight of 20,000 or less.

The composition according to the present invention is stable and capable of forming a polyion complex and providing good spreadability during application and improved texture such as reduced stickiness after application.

The composition according to the present invention is stable such that it can be homogeneous after the preparation thereof.

The composition according to the present invention can form a polyion complex with the (a) cationic polymer and the (b) anionic polymer, as well as the (c) non-polymeric acid having two or more pKa values or salt(s) thereof, as a crosslinker. The polyion complex may be in the form of a particle.

The composition according to the present invention can have good spreadability during application. Therefore, the composition according to the present invention can be easily spread during application on, for example, a keratin substance such as skin.

The composition according to the present invention can provide improved texture such as reduced stickiness, enhanced moisturizing feeling, enhanced softness and enhanced smoothness, after application.

The improvements in stickiness, moisturizing feeling, softness and smoothness, after application can be provided by a film formed by the composition according to the present invention.

Hereinafter, the composition, process, and the like according to the present invention will be explained in a more detailed manner.

### [Composition]

### (Cationic Polymer)

The composition according to the present invention includes (a) at least one cationic polymer.

There is no limit to the type of the (a) cationic polymer. Two or more different types of cationic polymers may be used in combination. Thus, a single type of cationic polymer or a combination of different types of cationic polymers may be used.

A cationic polymer has a positive charge density. The charge density of the (a) cationic polymer may be from 0.01 meq/g to 20 meq/g, preferably from 0.05 to 15 meq/g, and more preferably from 0.1 to 10 meq/g.

The (a) cationic polymer is selected from chitosans with a molecular weight of 20,000 or less, preferably 10,000 or less, and more preferably 7,000 or less.

Unless otherwise defined in the descriptions, "molecular weight" means a weight average molecular weight.

It may be preferable that the molecular weight of the (a) cationic polymer be 1,000 or more, preferably 1,200 or more, and more preferably 1,500 or more.

Chitosans comprise chitosan, salts of chitosan such as chitosan hydrochloride, and glycosides of chitosan. It is preferable that the (a) cationic polymer be chitosan with a molecular weight of 20,000 or less.

Chitosan is well known. Chitosan can be a linear polysaccharide composed of randomly distributed β-(1→4)-linked D-glucosamine (deacetylated unit) and N-acetyl-D-glucosamine (acetylated unit).

The actylation degree of chitosan may be more than 3% to less than 10%, preferably from 3% to 8%, and more preferably from 3% to 5%.

Chitosan can be prepared by, for example, treating the chitin shells of shrimp and other crustaceans with an alkaline substance, such as sodium hydroxide. However, it is preferable that chitosan be derived from fungus such as *Aspergillus Niger* because the chitosan derived from fungus could be free from any allergen. Thus, it may be preferable that the chitosan be derived from fungus.

The amount of the (a) cationic polymer(s) in the composition according to the present invention may be 0.01% by weight or more, preferably 0.05% by weight or more, and more preferably 0.1 % by weight or more, relative to the total weight of the composition.

The amount of the (a) cationic polymer(s) in the composition according to the present invention may be 15% by weight or less, preferably 10% by weight or less, and more preferably 5% by weight or less, relative to the total weight of the composition.

The amount of the (a) cationic polymer(s) in the composition according to the present invention may be from 0.01% to 15% by weight, preferably from 0.05% to 10% by weight, and more preferably from 0.1% to 5% by weight, relative to the total weight of the composition.

### (Anionic Polymer)

The composition according to the present invention includes (b) at least one anionic polymer.

There is no limit to the type of the (b) anionic polymer. Two or more different types of anionic polymers may be used in combination. Thus, a single type of anionic polymer or a combination of different types of anionic polymers may be used.

The (e) anionic polymer(s) can form polyion complex(es) with the (a) cationic polymer(s).

An anionic polymer has a positive charge density. The charge density of the (b) anionic polymer may be from 0.1 meq/g to 20 meq/g, preferably from 1 to 15 meq/g, and more preferably from 4 to 10 meq/g if the (b) anionic polymer is a synthetic anionic polymer, and the average substitution degree of the (b) anionic polymer may be from 0.1 to 3.0, preferably from 0.2 to 2.7, and more preferably from 0.3 to 2.5 if the (b) anionic polymer is a natural anionic polymer.

It may be preferable that the molecular weight of the anionic polymer be 300 or more, preferably 1,000 or more, even more preferably 5,000 or more, even more preferably 10,000 or more, even more preferably 50,000 or more, even more preferably 100,000 or more, and even more preferably 1,000,000 or more.

Unless otherwise defined in the descriptions, "molecular weight" may mean a weight average molecular weight.

The (b) anionic polymer may have at least one negatively chargeable and/or negatively charged moiety selected from the group consisting of a sulfuric group, a sulfate group, a sulfonic group, a sulfonate group, a phosphoric group, a phosphate group, a phosphonic group, a phosphonate group, a carboxylic group, and a carboxylate group, and preferably selected from the group consisting of a carboxylic group and a carboxylate group.

The (b) anionic polymer may be a homopolymer or a copolymer. The term "copolymer" is understood to mean both copolymers obtained from two kinds of monomers and those obtained from more than two kinds of monomers, such as terpolymers obtained from three kinds of monomers.

The (b) anionic polymer may be selected from natural and synthetic anionic polymers.

The (b) anionic polymer may comprise at least one hydrophobic chain.

The anionic polymer which may comprise at least one hydrophobic chain may be obtained by copolymerization of a monomer (a) chosen from carboxylic acids comprising an α,β-ethylenic unsaturation (monomer a') and 2-acrylamido-2-methylpropanesulphonic acid (monomer a") with a non-surface-active monomer (b) comprising an ethylenic unsaturation other than (a) and/or a monomer (c) comprising an ethylenic unsaturation resulting from the reaction of an acrylic monomer comprising an α,β-monoethylenic unsaturation or of an isocyanate monomer comprising a monoethylenic unsaturation with a monohydric nonionic amphiphilic component or with a primary or secondary fatty amine.

Thus, the anionic polymer with at least one hydrophobic chain may be obtained by two synthetic routes:
- either by copolymerization of the monomers (a') and (c), or (a'), (b) and (c), or (a") and (c), or (a"), (b) and (c),
- or by modification (and in particular esterification or amidation) of a copolymer formed from the monomers (a') or from the monomers (a') and (b), or (a") and (b), by a monohydric nonionic amphiphilic compound or a primary or secondary fatty amine.

Mention may in particular be made, as 2-acrylamido-2-methylpropanesulphonic acid copolymers, of those disclosed in the article "Micelle formation of random copolymers of sodium 2-(acrylamido)-2-methylpropanesulfonate and nonionic surfactant macromonomer in water as studied by fluorescence and dynamic light scattering - Macromolecules, 2000, Vol. 33, No. 10 - 3694-3704" and in applications EP-A-0 750 899 and EP-A-1 069 172.

The carboxylic acid comprising an α,β-monoethylenic unsaturation constituting the monomer (a') can be chosen from numerous acids and in particular from acrylic acid, methacrylic acid, crotonic acid, itaconic acid and maleic acid. It is preferably acrylic or methacrylic acid.

The copolymer can comprise a monomer (b) comprising a monoethylenic unsaturation which does not have a surfactant property. The preferred monomers are those which give water-insoluble polymers when they are homopolymerized. They can be chosen, for example, from C₁-C₄ alkyl acrylates and methacrylates, such as methyl acrylate, ethyl acrylate, butyl acrylate or the corresponding methacrylates. The more particularly preferred monomers are methyl acrylate and ethyl acrylate. The other monomers which can be used are, for example, styrene, vinyltoluene, vinyl acetate, acrylonitrile and vinylidene chloride. Unreactive monomers are preferred, these monomers being those in which the single ethylenic group is the only group which is reactive under the polymerization conditions. However, monomers which comprise groups which react under the effect of heat, such as hydroxyethyl acrylate, can optionally be used.

The monomer (c) is obtained by reaction of an acrylic monomer comprising α,β-monoethylenic unsaturation, such as (a), or of an isocyanate monomer comprising monoethylenic unsaturation with a monohydric nonionic amphiphilic compound or a primary or secondary fatty amine.

The monohydric nonionic amphiphilic compounds or the primary or secondary fatty amines used to produce the nonionic monomer (c) are well known. The monohydric nonionic amphiphilic compounds are generally alkoxylated hydrophobic compounds comprising an alkylene oxide forming the hydrophilic part of the molecule. The hydrophobic compounds are generally composed of an aliphatic alcohol or an alkylphenol, in which compounds a carbonaceous chain comprising at least six carbon atoms constitutes the hydrophobic part of the amphiphilic compound.

The preferred monohydric nonionic amphiphilic compounds are compounds having the following formula (V):

R-(OCH₂CHR')ₘ-(OCH₂CH₂)ₙ-OH (V)

in which R is chosen from alkyl or alkylene groups comprising from 6 to 30 carbon atoms and alkylaryl groups having alkyl radicals comprising from 8 to 30 carbon atoms, R' is chosen from alkyl groups comprising from 1 to 4 carbon atoms, n is a mean number ranging from approximately 1 to 150 and m is a mean number ranging from approximately 0 to 50, provided that n is at least as great as m.

Preferably, in the compounds of formula (V), the R group is chosen from alkyl groups comprising from 12 to 26 carbon atoms and alkylphenyl groups in which the alkyl group is C₈-C₁₃; the R' group is the methyl group; m = 0 and n = 1 to 25.

The preferred primary and secondary fatty amines are composed of one or two alkyl chains comprising from 6 to 30 carbon atoms.

The monomer used to form the nonionic urethane monomer (c) can be chosen from highly varied compounds. Use may be made of any compound comprising a copolymerizable unsaturation, such as an acrylic, methacrylic or allylic unsaturation. The monomer (c) can be obtained in particular from an isocyanate comprising a monoethylenic unsaturation, such as, in particular, α,α-dimethyl-m-isopropenylbenzyl isocyanate.

The monomer (c) can be chosen in particular from acrylates, methacrylates or itaconates of oxyethylenated (1 to 50 EO) C₆-C₃₀ fatty alcohol, such as steareth-20 methacrylate, oxyethylenated (25 EO) behenyl methacrylate, oxyethylenated (20 EO) monocetyl itaconate, oxyethylenated (20 EO) monostearyl itaconate or the acrylate modified by polyoxyethylenated (25 EO) C₁₂-C₂₄ alcohols and from dimethyl-m-isopropenylbenzyl isocyanates of oxyethylenated (1 to 50 EO) C₆-C₃₀ fatty alcohol, such as, in particular, the dimethyl-m-isopropenylbenzyl isocyanate of oxyethylenated behenyl alcohol.

According to a specific embodiment of the present invention, the anionic polymer is chosen from acrylic terpolymers obtained from (a) a carboxylic acid comprising an α,β-ethylenic unsaturation, (b) a non-surface-active monomer comprising an ethylenic unsaturation other than (a), and (c) a nonionic urethane monomer which is the reaction product of a monohydric nonionic amphiphilic compound with an isocyanate comprising a monoethylenic unsaturation.

Mention may in particular be made, as anionic polymers comprising at least one hydrophobic chain, of the acrylic acid/ethyl acrylate/alkyl acrylate terpolymer, such as the product as a 30% aqueous dispersion sold under the name Acusol 823 by Rohm & Haas; the acrylates/steareth-20 methacrylate copolymer, such as the product sold under the name Aculyn 22 by Rohm & Haas; the (meth)acrylic acid/ethyl acrylate/oxyethylenated (25 EO) behenyl methacrylate terpolymer, such as the product as an aqueous emulsion sold under the name Aculyn 28 by Rohm & Haas; the acrylic acid/oxyethylenated (20 EO) monocetyl itaconate copolymer, such as the product as a 30% aqueous dispersion sold under the name Structure 3001 by National Starch; the acrylic acid/oxyethylenated (20 EO) monostearyl itaconate copolymer, such as the product as a 30% aqueous dispersion sold under the name Structure 2001 by National Starch; the acrylates/acrylate modified by polyoxyethylenated (25 EO) C₁₂-C₂₄ alcohol copolymer, such as the 30-32% copolymer latex sold under the name Synthalen W2000 by 3V SA; or the methacrylic acid/methyl acrylate/dimethyl-meta-isopropenylbenzyl isocyanate of ethoxylated behenyl alcohol terpolymer, such as the product as a 24% aqueous dispersion and comprising 40 ethylene oxide groups disclosed in the document EP-A-0 173 109.

The (e) anionic polymers may also be Polyester-5, such as the product sold under the name of Eastman AQ^{™} 55S Polymer by EASTMAN CHEMICAL having the chemical formula below. 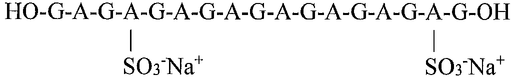
A: dicarboxylic acid moiety
G: glycol moiety
SO₃⁻Na⁺: sodium sulfo group
OH: hydroxyl group

It may be preferable that the (e) anionic polymer be selected from the group consisting of polysaccharides such as alginic acid, hyaluronic acid, and cellulose polymers (e.g., carboxymethylcellulose), anionic (co)polyaminoacids such as (co)polyglutamic acids, (co)poly(meth)acrylic acids, (co)polyamic acids, (co)polystyrene sulfonate, (co)poly(vinyl sulfate), dextran sulfate, chondroitin sulfate, (co)polymaleic acids, (co)polyfumaric acids, maleic acid (co)polymers, and salts thereof.

The maleic acid copolymer may comprise one or more maleic acid comonomers, and one or more comonomers chosen from vinyl acetate, vinyl alcohol, vinylpyrrolidone, olefins comprising from 2 to 20 carbon atoms, and styrene.

Thus, the "maleic acid copolymer" is understood to mean any polymer obtained by copolymerization of one or more maleic acid comonomers and of one or more comonomers chosen from vinyl acetate, vinyl alcohol, vinylpyrrolidone, olefins comprising from 2 to 20 carbon atoms, such as octadecene, ethylene, isobutylene, diisobutylene or isooctylene, and styrene, the maleic acid comonomers optionally being partially or completely hydrolysed. Use will preferably be made of hydrophilic polymers, that is to say polymers having a solubility in water of greater than or equal to 2 g/l.

In an advantageous aspect of the present invention, the maleic acid copolymer may have a molar fraction of maleic acid units of between 0.1 and 1, more preferably between 0.4 and 0.9.

The weight-average molar mass of the maleic acid copolymer may be between 1,000 and 500,000, and preferably between 1,000 and 50,000.

It is preferable that the maleic acid copolymer be a styrene/maleic acid copolymer, and more preferably sodium styrene/maleic acid copolymer.

Use will preferably be made of a copolymer of styrene and of maleic acid in a 50/50 ratio.

Use may be made, for example, of the styrene/maleic acid (50/50) copolymer, in the form of an ammonium salt at 30% in water, sold under the reference SMA1000H^{®} by Cray Valley or the styrene/maleic acid (50/50) copolymer, in the form of a sodium salt at 40% in water, sold under the reference SMA1000HNa^{®} by Cray Valley.

The use of the styrene/maleic acid copolymer such as sodium styrene/maleic acid copolymer may improve the wettability of a film prepared by the composition according to the present invention.

According to one embodiment of the present invention, it is preferable that the (e) anionic polymer be selected from hyaluronic acid and derivatives thereof.

Hyaluronic acid can be represented by the following chemical formula.

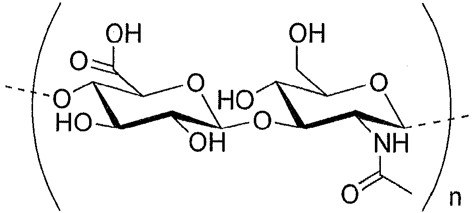

In the context of the present invention, the term "hyaluronic acid" covers in particular the basic unit of hyaluronic acid of formula:

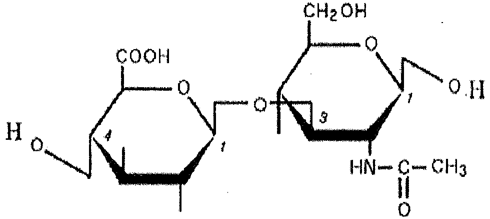

This is the smallest fraction of hyaluronic acid comprising a disaccharide dimer, namely D-glucuronic acid and N-acetylglucosamine.

The term "hyaluronic acid and derivatives thereof" also comprises, in the context of the present invention, the linear polymer comprising the polymeric unit described above, linked together in the chain via alternating β(1,4) and β(1,3) glycosidic linkages, having a molecular weight (MW) that can range between 380 and 13,000,000 daltons. This molecular weight depends in large part on the source from which the hyaluronic acid is obtained and/or on the preparation methods.

The term "hyaluronic acid and derivatives thereof" also comprises, in the context of the present invention, hyaluronic acid salts. As the salts, mention may be made of alkaline metal salts such as sodium salts and potassium salts, alkaline earth metal salts such as magnesium salts, ammonium salts, and mixtures thereof.

In the natural state, hyaluronic acid is present in pericellular gels, in the base substance of the connective tissues of vertebrate organs such as the dermis and epithelial tissues, and in particular in the epidermis, in the synovial fluid of the joints, in the vitreous humor, in the human umbilical cord and in the crista galli apophysis.

Thus, the term "hyaluronic acid and derivatives thereof" comprises all the fractions or subunits of hyaluronic acid having a molecular weight in particular within the molecular weight range recalled above.

In the context of the present invention, hyaluronic acid fractions which do not have inflammatory activity are preferably used.

By way of illustration of the various hyaluronic acid fractions, reference may be made to the document "Hyaluronan fragments: an information-rich system", R. Stern et al., European Journal of Cell Biology 58 (2006) 699-715, which reviews the listed biological activities of hyaluronic acid according to its molecular weight.

According to a preferred embodiment of the present invention, the hyaluronic acid fractions suitable for the use covered by the present invention have a molecular weight of between 50,000 and 5,000,000, in particular between 100,000 and 5,000,000, especially between 400,000 and 5,000,000 Da. In this case, the term used is high-molecular-weight hyaluronic acid.

Alternatively, the hyaluronic acid fractions that may also be suitable for the use covered by the present invention have a molecular weight of between 50,000 and 400,000 Da. In this case, the term used is intermediate-molecular-weight hyaluronic acid.

Alternatively again, the hyaluronic acid fractions that may be suitable for the use covered by the present invention have a molecular weight of less than 50,000 Da. In this case, the term used is low-molecular-weight hyaluronic acid.

Finally, the term "hyaluronic acid and derivatives thereof" also comprises hyaluronic acid esters in particular those in which all or some of the carboxylic groups of the acid functions are esterified with oxyethylenated alkyls or alcohols, containing from 1 to 20 carbon atoms, in particular with a degree of substitution at the level of the D-glucuronic acid of the hyaluronic acid ranging from 0.5 to 50%.

Mention may in particular be made of methyl, ethyl, n-propyl, n-pentyl, benzyl and dodecyl esters of hyaluronic acid. Such esters have in particular been described in D. Campoccia et al. "Semisynthetic resorbable materials from hyaluronan esterification", Biomaterials 19 (1998) 2101-2127.

The hyaluronic acid derivative may be, for example, acetylated hyaluronic acid or a salt thereof.

The molecular weights indicated above are also valid for the hyaluronic acid esters.

The hyaluronic acid may in particular be hyaluronic acid supplied by the company Hyactive under the trade name CPN (MW: 10 to 150 kDa), by the company Soliance under the trade name Cristalhyal (MW: 1.1.times.10⁶), by the company Bioland under the name Nutra HA (MW: 820 000 Da), by the company Bioland under the name Nutra AF (MW: 69 000 Da), by the company Bioland under the name Oligo HA (MW: 6100 Da) or else by the company Vam Farmacos Metica under the name D Factor (MW: 380 Da).

The (b) anionic polymer may be selected from polysaccharides, preferably hyaluronic acid and derivatives thereof, cellulose polymers and salts thereof, and a mixture thereof, and more preferably hyaluronic acid and salts thereof (e.g., sodium hyaluronate), carboxymethylcellulose and salts thereof, and a mixture thereof.

The amount of the (b) anionic polymer(s) in the composition according to the present invention may be 0.01% by weight or more, preferably 0.05% by weight or more, and more preferably 0.1% by weight or more, relative to the total weight of the composition.

The amount of the (b) anionic polymer(s) in the composition according to the present invention may be 15% by weight or less, preferably 10% by weight or less, and more preferably 5% by weight or less, relative to the total weight of the composition.

The amount of the (b) anionic polymer(s) in the composition according to the present invention may be from 0.01% to 15% by weight, preferably from 0.05% to 10% by weight, and more preferably from 0.1% to 5% by weight, relative to the total weight of the composition.

### (Non-Polymeric Acid Having Two or More Acid Dissociation Constants)

The composition according to the present invention includes (c) at least one non-polymeric acid having two or more pKa values or salt(s) thereof, i.e., at least one non-polymeric acid having two or more acid dissociation constants or salt(s) thereof. The pKa value (acid dissociation constant) is well known to those skilled in the art, and should be determined at a constant temperature such as 25°C.

The (c) non-polymeric acid having two or more pKa values or salt(s) thereof can function as a crosslinker for the (a) cationic polymer. The (a) cationic polymer(s) can be ionically-crosslinked by the (c) non-polymeric acid(s) having two or more pKa values or salt(s) thereof.

The (c) non-polymeric acid having two or more pKa values can be included in a polyion complex which can be formed by the (a) cationic polymer and the (b) anionic polymer. The (c) non-polymeric acid having two or more pKa values can influence on the polyion complex formed by the (a) cationic polymer and the (b) anionic polymer.

The term "non-polymeric" here means that the acid is not obtained by polymerizing two or more monomers. Therefore, the non-polymeric acid does not correspond to an acid obtained by polymerizing two or more monomers such as polycarboxylic acid.

It is preferable that the molecular weight of the (c) non-polymeric acid having two or more pKa values or salt(s) thereof is 1,000 or less, preferably 800 or less, and more preferably 700 or less.

There is no limit to the type of the (c) non-polymeric acid having two or more pKa values or salt(s) thereof. Two or more different types of non-polymeric acids having two or more pKa values or salts thereof may be used in combination. Thus, a single type of a non-polymeric acid having two or more pKa values or a salt thereof or a combination of different types of non-polymeric acids having two or more pKa values or salts thereof may be used.

The term "salt" here means a salt formed by addition of suitable base(s) to the non-polymeric acid having two or more pKa values, which may be obtained from a reaction with the non-polymeric acid having two or more pKa values with the base(s) according to methods known to those skilled in the art. As the salt, mention may be made of metal salts, for example salts with alkaline metal such as Na and K, and salts with alkaline earth metal such as Mg and Ca, and ammonium salts.

The (c) non-polymeric acid having two or more pKa values or salt(s) thereof may be an organic acid or salt(s) thereof, and preferably a hydrophilic or water-soluble organic acid or salt(s) thereof.

The (c) non-polymeric acid having two or more pKa values may have at least two acid groups selected from the group consisting of a carboxylic group, a sulfuric group, a sulfonic group, a phosphoric group, a phosphonic group, a phenolic hydroxyl group, and a mixture thereof.

The (c) non-polymeric acid having two or more pKa values may be a non-polymeric polyvalent acid.

The (c) non-polymeric acid having two or more pKa values may be selected from the group consisting of dicarboxylic acids, disulfonic acids, and diphosphoric acids, and a mixture thereof.

The (c) non-polymeric acid having two or more pKa values or salt(s) thereof may be selected from the group consisting of oxalic acid, malonic acid, succinic acid, glutaric acid, adipic acid, pimelic acid, suberic acid, azelaic acid, sebacic acid, fumaric acid, maleic acid, malic acid, citric acid, aconitic acid, oxaloacetic acid, tartaric acid, and salts thereof; aspartic acid, glutamic acid, and salts thereof; terephthalylidene dicamphor sulfonic acid or salts thereof (Mexoryl SX), Benzophenone-9; phytic acid, and salts thereof; Red 2 (Amaranth), Red 102 (New Coccine), Yellow 5 (Tartrazine), Yellow 6 (Sunset Yellow FCF), Green 3 (Fast Green FCF), Blue 1 (Brilliant Blue FCF), Blue 2 (Indigo Carmine), Red 201 (Lithol Rubine B), Red 202 (Lithol Rubine BCA), Red 204 (Lake Red CBA), Red 206 (Lithol Red CA), Red 207 (Lithol Red BA), Red 208 (Lithol Red SR), Red 219 (Brilliant Lake Red R), Red 220 (Deep Maroon), Red 227 (Fast Acid Magenta), Yellow 203 (Quinoline Yellow WS), Green 201 (Alizanine Cyanine Green F), Green 204 (Pyranine Conc), Green 205 (Light Green SF Yellowish), Blue 203 (Patent Blue CA), Blue 205 (Alfazurine FG), Red 401 (Violamine R), Red 405 (Permanent Re F5R), Red 502 (Ponceau 3R), Red 503 (Ponceau R), Red 504 (Ponceau SX), Green 401 (Naphthol Green B), Green 402 (Guinea Green B), and Black 401 (Naphthol Blue Black); folic acid, ascorbic acid, erythorbic acid, and salts thereof; cystine and salts thereof; EDTA and salts thereof; glycyrrhizin and salts thereof; and a mixture thereof.

It may be preferable that the (c) non-polymeric acid having two or more pKa values or salt(s) thereof be selected from the group consisting of terephthalylidene dicamphor sulfonic acid and salts thereof (Mexoryl SX), Yellow 6 (Sunset Yellow FCF), ascorbic acid, phytic acid and salts thereof, and a mixture thereof.

It may be more preferable that the (c) non-polymeric acid having two or more pKa values or salt(s) thereof be selected from the group consisting of terephthalylidene dicamphor sulfonic acid and salts thereof (Mexoryl SX), phytic acid and salts thereof, and a mixture thereof.

The amount of the (c) non-polymeric acid having two or more pKa values or salt(s) thereof in the composition according to the present invention may be 0.001% by weight or more, preferably 0.005% by weight or more, and more preferably 0.01% by weight or more, relative to the total weight of the composition.

The amount of the (c) non-polymeric acid having two or more pKa values or salt(s) thereof in the composition according to the present invention may be 10% by weight or less, preferably 5% by weight or less, and more preferably 1% by weight or less, relative to the total weight of the composition.

The amount of the (c) non-polymeric acid having two or more pKa values or salt(s) thereof in the composition according to the present invention may be from 0.001% to 10% by weight, preferably from 0.005% to 5% by weight, and more preferably from 0.01% to 1% by weight, relative to the total weight of the composition.

### (Water)

The composition according to the present invention comprises (d) water.

The amount of the (d) water may be 50% by weight or more, preferably 60% by weight or more, and more preferably 70% by weight or more, relative to the total weight of the composition.

The amount of the (d) water may be 99% by weight or less, preferably 98% by weight or less, and more preferably 97% by weight or less, relative to the total weight of the composition.

The amount of the (d) water may be from 50% to 99% by weight, preferably from 60% to 98% by weight, and more preferably from 70% to 97% by weight, relative to the total weight of the composition.

### (pH)

The pH of the composition according to the present invention may be from 3 to 9, preferably from 3.3 to 8.5, and more preferably from 3.5 to 8.

At a pH of from 3 to 9, the (a) anionic polymer and the (b) anionic polymer, as well as the (c) non-polymeric acid having two or more pKa values or salt(s) thereof, can form a stable polyion complex which may be in the form of a particle. Thus, the composition according to the present invention can be stable at a pH of from 3 to 9.

The pH of the composition according to the present invention may be adjusted by adding at least one alkaline agent and/or at least one acid, other than the (c) non-polymeric acid having two or more pKa values or salt(s) thereof. The pH of the composition according to the present invention may also be adjusted by adding at least one buffering agent.

### (Alkaline Agent)

The composition according to the present invention may comprise at least one alkaline agent. Two or more alkaline agents may be used in combination. Thus, a single type of alkaline agent or a combination of different types of alkaline agents may be used.

The alkaline agent may be an inorganic alkaline agent. It is preferable that the inorganic alkaline agent be selected from the group consisting of ammonia; alkaline metal hydroxides; alkaline earth metal hydroxides; alkaline metal phosphates and monohydrogenophosphates such as sodium phosphate or sodium monohydrogen phosphate.

As examples of the inorganic alkaline metal hydroxides, mention may be made of sodium hydroxide and potassium hydroxide. As examples of the alkaline earth metal hydroxides, mention may be made of calcium hydroxide and magnesium hydroxide. As an inorganic alkaline agent, sodium hydroxide is preferable.

The alkaline agent may be an organic alkaline agent. It is preferable that the organic alkaline agent be selected from the group consisting of monoamines and derivatives thereof; diamines and derivatives thereof; polyamines and derivatives thereof; basic amino acids and derivatives thereof; oligomers of basic amino acids and derivatives thereof; polymers of basic amino acids and derivatives thereof; urea and derivatives thereof; and guanidine and derivatives thereof.

As examples of the organic alkaline agents, mention may be made of alkanolamines such as mono-, di- and tri-ethanolamine, and isopropanolamine; urea, guanidine and their derivatives; basic amino acids such as lysine, ornithine or arginine; and diamines such as those described in the structure below: 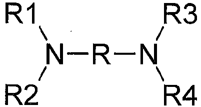 wherein R denotes an alkylene such as propylene optionally substituted by a hydroxyl or a C₁-C₄ alkyl radical, and R₁, R₂, R₃ and R₄ independently denote a hydrogen atom, an alkyl radical or a C₁-C₄ hydroxyalkyl radical, which may be exemplified by 1,3-propanediamine and derivatives thereof. Arginine, urea and monoethanolamine are preferable.

The alkaline agent(s) may be used in a total amount of from 0.01% to 15% by weight, preferably from 0.02% to 10% by weight, more preferably from 0.03% to 5% by weight, relative to the total weight of the composition, depending on their solubility.

### (Acid)

The composition according to the present invention may comprise at least one acid. Two or more acids may be used in combination. Thus, a single type of acid or a combination of different types of acids may be used.

As the acid, mention may be made of any inorganic or organic acids, preferably inorganic acids, which are commonly used in cosmetic products. A monovalent acid and/or a polyvalent acid may be used. A monovalent acid such as citric acid, lactic acid, sulfuric acid, phosphoric acid and hydrochloric acid (HCl) may be used. HCl is preferable.

The acid(s) may be used in a total amount of from 0.01% to 15% by weight, preferably from 0.02% to 10% by weight, more preferably from 0.03% to 5% by weight, relative to the total weight of the composition, depending on their solubility.

### (Buffering Agent)

The composition according to the present invention may comprise at least one buffering agent. Two or more buffering agents may be used in combination. Thus, a single type of buffering agent or a combination of different types of buffering agents may be used.

As the buffering agent, mention may be made of an acetate buffer (for example, acetic acid + sodium acetate), a phosphate buffer (for example, sodium dihydrogen phosphate + di-sodium hydrogen phosphate), a citrate buffer (for example, citric acid + sodium citrate), a borate buffer (for example, boric acid + sodium borate), a tartrate buffer (for example, tartaric acid + sodium tartrate dihydrate), Tris buffer (for example, tris(hydroxymethyl)aminomethane), and a Hepes buffer (4-(2-hydroxyethyl)-1-piperazineethanesulfonic acid).

### (Optional Additives)

The composition according to the present invention may comprise, in addition to the aforementioned components, components typically employed in cosmetics, specifically, oils, surfactants (in particular, nonionic surfactants) or emulsifiers, hydrophilic or lipophilic thickeners, organic volatile or non-volatile solvents, hydrophilic or hydrophobic UV filters, silicones and silicone derivatives, natural extracts derived from animals or vegetables, waxes, and the like, within a range which does not impair the effects of the present invention.

The composition according to the present invention may comprise the above optional additive(s) in an amount of from 0.01% to 50% by weight, preferably from 0.05% to 30% by weight, and more preferably from 0.1% to 10% by weight, relative to the total weight of the composition.

### (Preparation)

The composition according to the present invention can be prepared by mixing the essential ingredient(s) as explained above, and optional ingredient(s), if necessary, as explained above.

The method and means to mix the above essential and optional ingredients are not limited. Any conventional method and means can be used to mix the above essential and optional ingredients to prepare the composition according to the present invention.

### [Composition]

The composition according to the present invention may be intended to be used as a cosmetic composition. Thus, the cosmetic composition according to the present invention may be intended for application onto a keratin substance. Keratin substance here means a material containing keratin as a main constituent element, and examples thereof include the skin, scalp, nails, lips, hair, and the like. Thus, it is preferable that the cosmetic composition according to the present invention be used for a cosmetic process for the keratin substance, in particular skin.

Thus, the cosmetic composition according to the present invention may be a skin cosmetic composition, preferably a skin care composition or a skin makeup composition, and more preferably a skin care composition.

### [Film]

The composition according to the present invention may be used for preparing a film.

Thus, the present invention may also relate to a process for preparing a film, preferably a cosmetic film, optionally with a thickness of preferably more than 0.1 µm, more preferably 0.5 µm or more, and even more preferably 1.0 µm or more, comprising:
applying onto a substrate, preferably a keratin substance, more preferably skin, the composition according to the present invention; and
drying the composition.

The upper limit of the thickness of the above film is not limited. Thus, for example, the thickness of the above film may be 1 mm or less, preferably 500 µm or less, more preferably 300 µm or less, and even more preferably 100 µm or less.

Since the process for preparing a film which may relate to the present invention includes the steps of applying the composition according to the present invention onto a substrate, preferably a keratin substance, and more preferably skin, and of drying the composition, the process does not require any spin coating or spraying, and therefore, it is possible to easily prepare even a relatively thick film. Thus, the process for preparing a film which may relate to the present invention can prepare a film without any special equipment such as spin coaters and spraying machines.

Even if the film prepared by the above process is relatively thick, it is still thin and may be transparent, and therefore, may not be easy to perceive. Thus, the film can be used preferably as a cosmetic film.

If the substrate is not a keratin substance such as skin, the composition according to the present invention may be applied onto a substrate made from any material other than keratin. The materials of the non-keratinous substrate are not limited. Two or more materials may be used in combination. Thus, a single type of material or a combination of different types of materials may be used. In any event, it is preferable that the substrate be flexible or elastic.

If the substrate is not a keratin substance, it is preferable that the substrate be water-soluble because it is possible to leave the film by washing the substrate with water. As examples of the water-soluble materials, mention may be made of poly(meth) acrylic acids, polyethyleneglycols, polyacrylamides, polyvinylalcohol (PVA), starch, celluloseacetates, and the like. PVA is preferable.

If the non-keratinous substrate is in the form of a sheet, it may have a thickness of more than that of the film prepared by the above process, in order to ease the handling of the film attached to the substrate sheet. The thickness of the non-keratinous substrate sheet is not limited, but may be from 1 µm to 5 mm, preferably from 10 µm to 1 mm, and more preferably from 50 µm to 500 µm.

It is more preferable that the film prepared by the above process be releasable from the non-keratinous substrate. The mode of release is not limited. Therefore, the film prepared by the above process may be peeled from the non-keratinous substrate, or released by the dissolution of the substrate sheet into a solvent such as water.

Thus, the present invention may also relate to:
(1) A film, preferably a cosmetic film, optionally with a thickness of preferably more than 0.1 µm, more preferably 0.5 µm or more, and even more preferably 1.0 µm or more, prepared by a process comprising:
   applying onto a substrate, preferably a keratin substance, and more preferably skin, the composition according to the present invention; and
   drying the composition,
      and
(2) A film, preferably a cosmetic film, optionally with a thickness of preferably more than 0.1 µm, more preferably 0.5 µm or more, and even more preferably 1.0 µm or more, comprising:
   (a) at least one cationic polymer;
   (b) at least one anionic polymer; and
   (c) at least one non-polymeric acid having two or more pKa values or salt(s)
   thereof, wherein
   the (a) cationic polymer is selected from chitosans with a molecular weight of 20,000 or less.

The above explanations regarding the (a) cationic polymer, the (b) anionic polymer, and the (c) non-polymeric acid having two or more pKa values or salt(s) thereof can apply to those in the above films (1) and (2).

The film thus obtained above can be self-standing. The term "self-standing" here means that the film can be in the form of a sheet and can be handled as an independent sheet without the assistance of a substrate or support. Thus, the term "self-standing" may have the same meaning as "self-supporting".

It is preferable that the above film be hydrophobic.

The term "hydrophobic" in the present specification means that the solubility of the polymer in water (preferably with a volume of 1 liter) at from 20°C to 40°C, preferably from 25°C to 40°C, and more preferably from 30°C to 40°C is less than 10% by weight, preferably less than 5% by weight, more preferably less than 1% by weight, and even more preferably less than 0.1% by weight, relative to the total weight of the polymer. It is most preferable that the polymer is not soluble in water.

If the above film is hydrophobic, the film can have water-resistant properties, and therefore, it can remain on a keratin substance such as skin even if the surface of the keratin substance is wet due to, for example, sweat and rain. Thus, when the film provides any cosmetic effect, the cosmetic effect can last a long time.

On the other hand, the above film can be easily removed from a keratin substance such as skin under alkaline conditions such as a pH of from 8 to 12, preferably from 9 to 11. Therefore, the above film is difficult to remove with water, while it can be easily removed with a soap which can provide such alkaline conditions.

The above film may comprise at least one biocompatible and/or biodegradable polymer layer. Two or more biocompatible and/or biodegradable polymers may be used in combination. Thus, a single type of biocompatible and/or biodegradable polymer or a combination of different types of biocompatible and/or biodegradable polymers may be used.

The term "biocompatible" polymer in the present specification means that the polymer does not have excess interaction between the polymer and cells in the living body including the skin, and the polymer is not recognized by the living body as a foreign material.

The term "biodegradable" polymer in the present specification means that the polymer can be degraded or decomposed in a living body due to, for example, the metabolism of the living body itself or the metabolism of the microorganisms which may be present in the living body. Also, the biodegradable polymer can be degraded by hydrolysis.

If the above film includes a biocompatible and/or biodegradable polymer, it is less irritable or not irritable to the skin, and does not cause any rash. In addition, due to the use of a biocompatible and/or biodegradable polymer, the above film can adhere well to the skin.

The above film can be used for cosmetic treatments of keratin substances, preferably skin, in particular the face. The above film can be in any shape or form. For example, it can be used as a full-face mask sheet, or a patch for a part of the face such as the cheek, nose, and around the eyes.

### [Cosmetic Process and Use]

The present invention also relates to:
a cosmetic process for a keratin substance such as skin, comprising:
applying to the keratin substance the composition according to the present invention; and
drying the composition to form a cosmetic film on the keratin substance,
   and
a use of the composition according to the present invention for the preparation of a cosmetic film on a keratin substance such as skin.

The cosmetic process here means a non-therapeutic cosmetic method for caring for and/or making up the surface of a keratin substance such as skin.

In both the above process and use, the above cosmetic film is resistant to water with a pH of 7 or less, and is removable with water with a pH of more than 7, preferably 8 or more, and more preferably 9 or more.

In other words, the above cosmetic film can be water-resistant under neutral or acidic conditions such as a pH of 7 or less, preferably in a range of 6 or more and 7 or less, and more preferably in a range of 5 or more and 7 or less, while the above cosmetic film can be removed under alkaline conditions such as a pH of more than 7, preferably 8 or more, and more preferably 9 or more. The upper limit of the pH is preferably 13, more preferably 12, and even more preferably 11.

Accordingly, the above cosmetic film can be water-resistant, and therefore, it can remain on a keratin substance such as skin even if the surface of the keratin substance is wet due to, for example, sweat and rain. On the other hand, the above cosmetic film can be easily removed from a keratin substance such as skin under alkaline conditions. Therefore, the above cosmetic film is difficult to remove with water, while it can be easily removed with a soap which can provide alkaline conditions.

Furthermore, the above cosmetic film may have cosmetic effects such as mattifying a keratin substance such as skin, absorbing or adsorbing sebum or malodour and/or protecting the keratin substance from, for example, dirt or pollutants, due to the properties of the polyion complex in the cosmetic film, even if the cosmetic film does not include any cosmetic active ingredient.

In addition, the above cosmetic film may immediately change or modify the appearance of the skin by changing light reflection on the skin and the like, even if the cosmetic film does not include any cosmetic active ingredient. Therefore, it may be possible for the above cosmetic film to conceal skin defects such as pores or wrinkles. Further, the above cosmetic film may immediately change or modify the feeling to the touch of the skin by changing the surface roughness on the skin and the like. Furthermore, the above cosmetic film may immediately protect the skin by covering the surface of the skin and shielding the skin, as a barrier, from environmental stresses such as pollutants, contaminants and the like.

The above cosmetic effects can be adjusted or controlled by changing the chemical composition, the thickness and/or the surface roughness of the above cosmetic film.

If the above cosmetic film includes at least one additional cosmetic active ingredient, the cosmetic film can have cosmetic effects provided by the additional cosmetic active ingredient(s). For example, if the cosmetic film includes at least one cosmetic active ingredient selected from anti-aging agents, anti-sebum agents, deodorant agents, antiperspirant agents, whitening agents and a mixture thereof, the cosmetic film can treat the aging of the skin, absorbing sebum on the skin, controlling odors on the skin, controlling perspiration on the skin, and/or whitening of the skin.

It is also possible to apply a makeup cosmetic composition onto the above cosmetic film or sheet after it has been applied onto the skin.

The present invention may also relate to a use of (a) at least one cationic polymer, wherein the (a) cationic polymer is selected from chitosans with a molecular weight of 20,000 or less,
in a composition comprising:
   (b) at least one anionic polymer;
   (c) at least one non-polymeric acid having two or more pKa values or salt(s) thereof; and
   (d) water,
in order to stabilize the composition and/or improve the spreadability of the composition. The composition is stable such that it can be homogeneous after the preparation thereof. The composition can be easily spread during application on, for example, a keratin substance such as skin.

The present invention may also relate to a use of (a) at least one cationic polymer, wherein the (a) cationic polymer is selected from chitosans with a molecular weight of 20,000 or less,
in a film comprising:
   (b) at least one anionic polymer; and
   (c) at least one non-polymeric acid having two or more pKa values or salt(s) thereof,
in order to improve the texture of film, preferably in order to enhance moisturizing feeling, softness and smoothness provided by the film and/or to reduce stickiness of the film, and more preferably in order to reduce the stickiness of the film.

The above explanations regarding the (a) cationic polymer, the (b) anionic polymer, the (c) non-polymeric acid having two or more pKa values or salt(s) thereof, and the (d) water can apply to those in the above uses.

### EXAMPLES

The present invention will be described in a more detailed manner by way of examples. However, they should not be construed as limiting the scope of the present invention.

### [Examples 1-9 and Comparative Examples 1-5]

### [Preparations]

Each of the compositions according to Examples 1-9 and Comparative Examples 1-5 was prepared by mixing the ingredients shown in Tables 1 and 2.

The numerical values for the amounts of the ingredients in Tables 1 and 2 are all based on "% by weight" as raw materials.

The details of the preparations are as follows.

### (Example 1)

First, sodium hyaluronate was added into water and solubilized to prepare an aqueous solution of a polyanion. After solubilizing, an appropriate amount of sodium hydroxide was added to the aqueous solution of polyanion to make the aqueous solution alkaline. Next, as a polycation, chitosan with a molecular weight of 3,000 was added to the alkaline aqueous solution. The formation of polyion complex in the aqueous solution was avoided due to the alkaline condition of the aqueous solution. Next, an appropriate amount of phytic acid was added to the aqueous solution. A polyion complex in the form of particles was formed.

### (Examples 2 and 3)

The preparation steps for Example 1 was repeated with the proviso that the amounts of polyanion (sodium hyaluronate) and polycation (chitosan) were changed.

### (Example 4)

The preparation steps for Example 1 was repeated with the proviso that the amounts of polyanion and polycation were changed, and that chitosan with a molecular weight of between 10,000 and 20,000 was used instead of chitosan with a molecular weight of 3,000.

### (Example 5)

The preparation steps for Example 1 was repeated with the proviso that carboxymethylcellulose was used instead of sodium hyaluronate.

### (Example 6)

The preparation steps for Example 1 was repeated with the proviso that the molecular weight of chitosan was changed from 3,000 to 5,000.

### (Example 7)

The preparation steps for Example 1 was repeated with the proviso that the molecular weight of chitosan was changed from 3,000 to 7,000.

### (Example 8)

The preparation steps for Example 5 was repeated with the proviso that the molecular weight of chitosan was changed from 3,000 to 5,000.

### (Example 9)

The preparation steps for Example 5 was repeated with the proviso that the molecular weight of chitosan was changed from 3,000 to 7,000.

### (Comparative Example 1)

The preparation steps for Example 2 was repeated with the proviso that chitosan with a molecular weight of 100,000 or more was used instead of chitosan with a molecular weight of 3,000.

### (Comparative Example 2)

The preparation steps for Example 2 was repeated with the proviso that phytic acid and sodium hydroxide were not used.

### (Comparative Example 3)

The preparation steps for Example 2 was repeated with the proviso that the molecular weight of chitosan was changed from 3,000 to 5,000, and that phytic acid and sodium hydroxide were not used.

### (Comparative Example 4)

The preparation steps for Example 2 was repeated with the proviso that the molecular weight of chitosan was changed from 3,000 to 7,000, and that phytic acid and sodium hydroxide were not used.

### (Comparative Example 5)

The preparation steps for Example 1 was repeated with the proviso that the amount of polycation was changed, and that poly-ε-lysine was used instead of chitosan with a molecular weight of 3,000.

**Table 1**

| | Ex. 1 | Ex.2 | Ex. 3 | Ex. 4 | Ex. 5 | Ex. 6 | Ex. 7 | Ex. 8 | Ex. 9 |
|---|---|---|---|---|---|---|---|---|---|
| Water | qsp 100 | qsp 100 | qsp 100 | qsp 100 | qsp 100 | qsp 100 | qsp 100 | qsp 100 | qsp 100 |
| Sodium Hyaluronate | 1.0 | 0.2 | 2.0 | 0.1 | - | 1.0 | 1.0 | - | - |
| Carboxymethylcellulose | - | - | - | - | 1.0 | - | - | 1.0 | 1.0 |
| Chitosan (M.W. = 3,000) | 0.5 | 0.1 | 1.0 | - | 0.5 | - | - | - | - |
| Chitosan (M.W. = 5,000) | - | - | - | - | - | 0.5 | - | 0.5 | - |
| Chitosan (M.W. = 7,000) | - | - | - | - | - | - | 0.5 | - | 0.5 |
| Chitosan (10,000 ≤ M.W. ≤ 20,000) | - | - | - | 0.05 | - | - | - | - | - |
| Phytic Acid (50wt% solution) | 0.55 | 0.11 | 1.1 | 0.05 | 0.55 | 0.55 | 0.55 | 0.55 | 0.55 |
| Sodium Hydroxide | 0.29 | 0.06 | 0.58 | 0.14 | 0.29 | 0.29 | 0.29 | 0.29 | 0.29 |
| pH | 6.0 | 6.0 | 6.0 | 6.0 | 6.0 | 6.0 | 6.0 | 6.0 | 6.0 |
| Stability | Good | Good | Good | Good | Good | Good | Fair | Good | Fair |

| | | | | | | | | | |
|---|---|---|---|---|---|---|---|---|---|
| M.W.: Molecular Weight | | | | | | | | | |

**Table 2**

| | Comp. Ex. 1 | Comp. Ex. 2 | Comp. Ex. 3 | Comp. Ex. 4 | Comp. Ex. 5 |
|---|---|---|---|---|---|
| Water | qsp 100 | qsp 100 | qsp 100 | qsp 100 | qsp 100 |
| Sodium Hyaluronate | 0.2 | 0.2 | 0.2 | 0.2 | 1.0 |
| Chitosan (M.W. = 3,000) | - | 0.1 | - | - | - |
| Chitosan (M.W. = 5,000) | - | - | 0.1 | - | - |
| Chitosan (M.W. = 7,000) | - | - | - | 0.1 | - |
| Chitosan (100,000 ≤ M.W.) | 0.1 | - | - | - | - |
| Polyepsilon Lysine (25wt% solution) | - | - | - | - | 2.0 |
| Phytic Acid (50wt% solution) | 0.11 | - | - | - | 0.55 |
| Sodium Hydroxide | 0.06 | - | - | - | - |
| pH | 6.0 | 6.0 | 6.0 | 6.0 | 6.0 |
| Stability | Poor | Poor | Poor | Poor | Good |

| | | | | | |
|---|---|---|---|---|---|
| M.W.: Molecular Weight | | | | | |

### [Evaluations]

### (Stability)

The compositions according to Examples 1-9 and Comparative Examples 1-5 just after the preparation thereof were visually observed and evaluated in accordance with the following criteria.
Goode: homogeneous without precipitation
Fair: homogeneous without precipitation, but slowly precipitates after few days
Poor: inhomogeneous with precipitation

The results are shown in Tables 1 and 2.

### (Sensory Evaluations)

Three panelists evaluated each of the compositions according to Examples 1 and 5-9 as well as Comparative Example 5 at the timing of during and after application of the composition. Specifically, each panelist applied each composition on his or her hand and spread it, followed by drying, in order to evaluate spreadability, stickiness upon drying, moisturizing feeling after drying, softness after drying and smoothness after drying and graded from 1 (low) to 5 (high) in consideration of a control which is a 1wt% sodium hyaluronate (HA) or carboxymethylcellulose (CMC) aqueous solution. It was then classified in the following three categories based on the average of the grade:
Much Better: much better than control
Better: better than control
Same: same with control

The results are shown in Tables 3 and 4.

**Table 3**

| | Ex. 1 | Ex. 6 | Ex. 7 | Comp. Ex. 5 | HA 1% Solution |
|---|---|---|---|---|---|
| Spreadability | Better | Better | Better | Same | Control |
| Stickiness | Much Better | Much Better | Much Better | Better | Control |
| Moisturizing Feeling | Better | Better | Better | Better | Control |
| Softness | Better | Better | Better | Better | Control |
| Smoothness | Better | Better | Better | Better | Control |

**Table 4**

| | Ex. 5 | Ex. 8 | Ex. 9 | CMC 1% Solution |
|---|---|---|---|---|
| Spreadability | Better | Better | Better | Control |
| Stickiness | Better | Better | Better | Control |
| Moisturizing Feeling | Better | Better | Better | Control |
| Softness | Better | Better | Better | Control |
| Smoothness | Better | Better | Better | Control |

### (Summary)

The compositions according to Examples 1-9 were stable (cf. Table 1).

The spreadability of the compositions according to Examples 1 and 5-9 and the texture provided by the compositions according to Examples 1 and 5-9 were improved due to the formation of polyion complex by a combination of a cationic polymer (chitosan with a molecular weight of 20,000 or less) and an anionic polymer (sodium hyaluronate or carboxymethylcellulose), as compared to a composition (control) including only the anionic polymer (cf. Tables 3 and 4).

The results of sensory evaluations for Example 1 could also apply to Examples 2-4 because the composition according to Example 1 and the compositions according to Examples 2-4 are similar.

The composition according to Comparative Example 1 which included chitosan with a higher molecular weight was not stable (cf. Table 2).

The composition according to Comparative Example 2 which did not include phytic acid was not stable (cf. Table 2).

The composition according to Comparative Example 3 which did not include phytic acid was not stable (cf. Table 2).

The composition according to Comparative Example 4 which did not include phytic acid was not stable (cf. Table 2).

The composition according to Comparative Example 5 which included poly-ε-lysine instead of chitosan was stable but less spreadable during application and more sticky after application than the composition according to the present invention (cf. Tables 2 and 3). Thus, the composition according to the present invention was more spreadable during application and less sticky after application than the composition according to Comparative Example 5 due to the use of chitosan with a molecular weight of 20,000 or less instead of poly-ε-lysine (cf. Table 3).

## Claims

1. A composition, comprising:
(a) at least one cationic polymer:
(b) at least one anionic polymer;
(c) at least one non-polymeric acid having two or more pKa values or salt(s) thereof; and
(d) water,
wherein
the (a) cationic polymer is selected from chitosans with a molecular weight of 20,000 or less.

2. The composition according to Claim 1, wherein the amount of the (a) cationic polymer(s) in the composition is from 0.01% to 15% by weight, preferably from 0.05% to 10% by weight, and more preferably from 0.1% to 5% by weight, relative to the total weight of the composition.

3. The composition according to Claim 1 or 2, wherein the (b) anionic polymer has at least one negatively chargeable and/or negatively charged moiety selected from the group consisting of a sulfuric group, a sulfate group, a sulfonic group, a sulfonate group, a phosphoric group, a phosphate group, a phosphonic group, a phosphonate group, a carboxylic group, and a carboxylate group, and preferably selected from the group consisting of a carboxylic group and a carboxylate group.

4. The composition according to any one of Claims 1 to 3, wherein the (b) anionic polymer is selected from the group consisting of polysaccharides such as alginic acid, hyaluronic acid, and cellulose polymers, anionic (co)polyaminoacids such as (co)polyglutamic acids, (co)poly(meth)acrylic acids, (co)polyamic acids, (co)polystyrene sulfonate, (co)poly(vinyl sulfates), dextran sulfate, chondroitin sulfate, (co)polymaleic acids, polyfumaric acids, maleic acid (co)polymers, and salts thereof.

5. The composition according to any one of Claims 1 to 4, wherein the (b) anionic polymer is selected from polysaccharides, preferably hyaluronic acid and salts and esters thereof, cellulose polymers and salts thereof, and a mixture thereof, and more preferably hyaluronic acid and salts thereof, carboxymethylcellulose and salts thereof, and a mixture thereof.

6. The composition according to any one of Claims 1 to 5, wherein the amount of the (b) anionic polymer(s) in the composition is from 0.01% to 15% by weight, preferably from 0.05% to 10% by weight, and more preferably from 0.1% to 5% by weight, relative to the total weight of the composition.

7. The composition according to any one of Claims 1 to 6, wherein the (c) non-polymeric acid having two or more pKa values or salt(s) thereof is an organic acid or salt(s) thereof, preferably a hydrophilic or water-soluble organic acid or salt(s) thereof, and more preferably phytic acid or salts thereof, terephthalylidene dicamphor sulfonic acid or salts thereof, or a mixture thereof.

8. The composition according to any one of Claims 1 to 7, wherein the amount of the (c) non-polymeric acid having two or more pKa values or salt(s) thereof in the composition is from 0.001% to 10% by weight, preferably from 0.005% to 5% by weight, and more preferably from 0.01% to 1% by weight, relative to the total weight of the composition.

9. The composition according to any one of Claims 1 to 8, wherein the amount of the (d) water in the composition is from 50% to 99% by weight, preferably from 60% to 98% by weight, and more preferably from 70% to 97% by weight, relative to the total weight of the composition.

10. The composition according to any one of Claims 1 to 9, wherein the pH of the composition is from 3 to 9, preferably from 3.5 to 8.5, and more preferably from 4 to 8.

11. The composition according to any one of Claims 1 to 10, wherein the composition is a cosmetic composition, preferably a skin cosmetic composition, and more preferably a skin care cosmetic composition.

12. A cosmetic process for a keratin substance such as skin, comprising
applying to the keratin substance the composition according to any one of Claims 1 to 11; and
drying the composition to form a cosmetic film on the keratin substance.

13. A use of (a) at least one cationic polymer, wherein the (a) cationic polymer is selected from chitosans with a molecular weight of 20,000 or less,
in a composition comprising:
(b) at least one anionic polymer;
(c) at least one non-polymeric acid having two or more pKa values or salt(s) thereof; and
(d) water,
in order to stabilize the composition and/or improve the spreadability of the composition.

14. A film, preferably a cosmetic film, comprising:
(a) at least one cationic polymer;
(b) at least one anionic polymer; and
(c) at least one non-polymeric acid having two or more pKa values or salt(s) thereof,
wherein
the (a) cationic polymer is selected from chitosans with a molecular weight of 20,000 or less.

15. A use of (a) at least one cationic polymer, wherein the (a) cationic polymer is selected from chitosans with a molecular weight of 20,000 or less,
in a film comprising:
(b) at least one anionic polymer; and
(c) at least one non-polymeric acid having two or more pKa values or salt(s) thereof,
in order to improve the texture of film, preferably in order to enhance moisturizing feeling, softness and smoothness provided by the film and/or to reduce stickiness of the film, and more preferably in order to reduce the stickiness of the film.

## Patentansprüche

1. Zusammensetzung, umfassend:
(a) wenigstens ein kationisches Polymer;
(b) wenigstens ein anionisches Polymer;
(c) wenigstens eine nichtpolymere Säure mit zwei oder mehr pKa-Werten oder Salz(e) davon; und
(d) Wasser,
wobei
das (a) kationische Polymer ausgewählt ist aus Chitosanen mit einem Molekulargewicht von 20.000 oder weniger.

2. Zusammensetzung nach Anspruch 1, wobei die Menge der (a) kationischen Polymer(e) in der Zusammensetzung von 0,01 Gew.-% bis 15 Gew.-%, vorzugsweise von 0,05 Gew.-% bis 10 Gew.-% und bevorzugter von 0,1 Gew.-% bis 5 Gew.-% beträgt, bezogen auf das Gesamtgewicht der Zusammensetzung.

3. Zusammensetzung nach Anspruch 1 oder 2, wobei das (b) anionische Polymer wenigstens eine negativ ladbare und/oder negativ geladene Einheit ausgewählt aus der Gruppe bestehend aus einer Schwefelsäuregruppe, einer Sulfatgruppe, einer Sulfonsäuregruppe, einer Sulfonatgruppe, einer Phosphorsäuregruppe, einer Phosphatgruppe, einer Phosphonsäuregruppe, einer Phosphonatgruppe, einer Carboxylgruppe und einer Carboxylatgruppe aufweist und vorzugsweise ausgewählt ist aus der Gruppe bestehend aus einer Carboxylgruppe und einer Carboxylatgruppe.

4. Zusammensetzung nach einem der Ansprüche 1 bis 3, wobei das (b) anionische Polymer ausgewählt ist aus der Gruppe bestehend aus Polysacchariden, wie Alginsäure, Hyaluronsäure, und Cellulosepolymeren, anionischen (Co)Polyaminosäuren, wie (Co)Polyglutaminsäuren, (Co)Poly(meth)acrylsäuren, (Co)Polyamidsäuren, (Co) Polystyrolsulfonat, (Co)Poly(vinylsulfaten), Dextransulfat, Chondroitinsulfat, (Co)Polymaleinsäuren, Polyfumarsäuren, Maleinsäure(co)polymeren und deren Salzen.

5. Zusammensetzung nach einem der Ansprüche 1 bis 4, wobei das (b) anionische Polymer ausgewählt ist aus Polysacchariden, vorzugsweise Hyaluronsäure und Salzen und Estern davon, Cellulosepolymeren und Salzen davon und einer Mischung davon und bevorzugter Hyaluronsäure und Salzen davon, Carboxymethylcellulose und Salzen davon und einer Mischung davon.

6. Zusammensetzung nach einem der Ansprüche 1 bis 5, wobei die Menge der (b) anionischen Polymer(e) in der Zusammensetzung von 0,01 Gew.-% bis 15 Gew.-%, vorzugsweise von 0,05 Gew.-% bis 10 Gew.-% und bevorzugter von 0,1 Gew.-% bis 5 Gew.-% beträgt, bezogen auf das Gesamtgewicht der Zusammensetzung.

7. Zusammensetzung nach einem der Ansprüche 1 bis 6, wobei die (c) nichtpolymere Säure mit zwei oder mehr pKa-Werten oder Salz(e) davon eine organische Säure oder Salz(e) davon ist, vorzugsweise eine hydrophile oder wasserlösliche organische Säure oder Salz(e) davon und bevorzugter Phytinsäure oder Salze davon, Terephthalylidendicamphersulfonsäure oder Salze davon oder eine Mischung davon.

8. Zusammensetzung nach einem der Ansprüche 1 bis 7, wobei die Menge der (c) nichtpolymeren Säure mit zwei oder mehr pKa-Werten oder Salz(en) davon in der Zusammensetzung von 0,001 Gew.-% bis 10 Gew.-%, vorzugsweise von 0,005 Gew.-% bis 5 Gew.-% und bevorzugter von 0,01 Gew.-% bis 1 Gew.-% beträgt, bezogen auf das Gesamtgewicht der Zusammensetzung.

9. Zusammensetzung nach einem der Ansprüche 1 bis 8, wobei die Menge des (d) Wassers in der Zusammensetzung von 50 Gew.-% bis 99 Gew.-%, vorzugsweise von 60 Gew.-% bis 98 Gew.-% und bevorzugter von 70 Gew.-% bis 97 Gew.-% beträgt, bezogen auf das Gesamtgewicht der Zusammensetzung.

10. Zusammensetzung nach einem der Ansprüche 1 bis 9, wobei der pH-Wert der Zusammensetzung von 3 bis 9, vorzugsweise 3,5 bis 8,5 und bevorzugter 4 bis 8 beträgt.

11. Zusammensetzung nach einem der Ansprüche 1 bis 10, wobei die Zusammensetzung eine kosmetische Zusammensetzung, vorzugsweise eine hautkosmetische Zusammensetzung und bevorzugter eine kosmetische Hautpflegezusammensetzung ist.

12. Kosmetisches Verfahren für eine Keratinsubstanz, wie Haut, umfassend Aufbringen der Zusammensetzung nach einem der Ansprüche 1 bis 11 auf die Keratinsubstanz; und Trocknen der Zusammensetzung, um einen kosmetischen Film auf der Keratinsubstanz zu bilden.

13. Verwendung von (a) mindestens einem kationischen Polymer, wobei das (a) kationische Polymer ausgewählt ist aus Chitosanen mit einem Molekulargewicht von 20.000 oder weniger,
in einer Zusammensetzung, umfassend:
(b) wenigstens ein anionisches Polymer;
(c) wenigstens eine nichtpolymere Säure mit zwei oder mehr pKa-Werten oder Salz(e) davon; und
(d) Wasser,
um die Zusammensetzung zu stabilisieren und/oder die Spreadbarkeit der Zusammensetzung zu verbessern.

14. Film, vorzugsweise ein kosmetischer Film, umfassend:
(a) wenigstens ein kationisches Polymer;
(b) wenigstens ein anionisches Polymer und
(c) wenigstens eine nichtpolymere Säure mit zwei oder mehr pKa-Werten oder Salz(e) davon,
wobei
das (a) kationische Polymer ausgewählt ist aus Chitosanen mit einem Molekulargewicht von 20.000 oder weniger.

15. Verwendung von (a) mindestens einem kationischen Polymer, wobei das (a) kationische Polymer ausgewählt ist aus Chitosanen mit einem Molekulargewicht von 20.000 oder weniger,
in einem Film, umfassend:
(b) wenigstens ein anionisches Polymer und
(c) wenigstens eine nichtpolymere Säure mit zwei oder mehr pKa-Werten oder Salz(e) davon,
um die Textur des Films zu verbessern, vorzugsweise um das Feuchtigkeitsgefühl, die Weichheit und Glätte zu verbessern, die der Film bereitstellt, und/oder die Klebrigkeit des Films zu reduzieren, und bevorzugter, um die Klebrigkeit des Films zu reduzieren.

## Revendications

1. Composition, comprenant :
(a) au moins un polymère cationique ;
(b) au moins un polymère anionique ;
(c) au moins un acide non polymérique ayant au moins deux valeurs de pKa ou un ou plusieurs sels de celui-ci ; et
(d) de l'eau ;
dans laquelle
le polymère cationique (a) est choisi parmi les chitosanes ayant un poids moléculaire de 20 000 ou moins.

2. Composition selon la revendication 1, dans laquelle la quantité du ou des polymères cationiques (a) dans la composition est de 0,01 % à 15 % en poids, préférablement de 0,05 % à 10 % en poids, et plus préférablement de 0,1 % à 5 % en poids, par rapport au poids total de la composition.

3. Composition selon la revendication 1 ou 2, dans laquelle le polymère anionique (b) a au moins un groupement chargeable négativement et/ou négativement chargé choisi dans le groupe constitué par un groupe sulfurique, un groupe sulfate, un groupe sulfonique, un groupe sulfonate, un groupe phosphorique, un groupe phosphate, un groupe phosphonique, un groupe phosphonate, un groupe carboxylique et un groupe carboxylate, et de préférence choisi dans le groupe constitué par un groupe carboxylique et un groupe carboxylate.

4. Composition selon l'une quelconque des revendications 1 à 3, dans laquelle le polymère anionique (b) est choisi dans le groupe constitué des polysaccharides tels que l'acide alginique, l'acide hyaluronique et les polymères de cellulose, les (co)polyaminoacides anioniques tels que les acides (co)polyglutamiques, les acides (co)poly(méth)acryliques, les acides (co)polyamiques, le sulfonate de (co)polystyrène, les (co)poly(vinyl sulfates), le sulfate de dextrane, le sulfate de chondroïtine, les acides (co)polymaléiques, les acides polyfumariques, les (co)polymères d'acide maléique et leurs sels.

5. Composition selon l'une quelconque des revendications 1 à 4, dans laquelle le polymère anionique (b) est choisi parmi les polysaccharides, de préférence l'acide hyaluronique et ses sels et esters, les polymères de cellulose et ses sels, et un mélange de ceux-ci, et plus préférablement l'acide hyaluronique et ses sels, la carboxyméthylcellulose et ses sels, et un mélange de ceux-ci.

6. Composition selon l'une quelconque des revendications 1 à 5, dans laquelle la quantité du ou des polymères anioniques (b) dans la composition est de 0,01 % à 15 % en poids, préférablement de 0,05 % à 10 % en poids, et plus préférablement de 0,1 % à 5 % en poids, par rapport au poids total de la composition.

7. Composition selon l'une quelconque des revendications 1 à 6, dans laquelle l'acide non polymérique (c) ayant au moins deux valeurs de pKa ou un ou plusieurs sels de celui-ci est un acide organique ou un ou plusieurs sels de celui-ci, de préférence un acide organique hydrophile ou hydrosoluble ou un ou plusieurs sels de celui-ci, et plus préférablement l'acide phytique ou des sels de celui-ci, l'acide téréphtalylidènedicamphresulfonique ou ses sels, ou un mélange de ceux-ci.

8. Composition selon l'une quelconque des revendications 1 à 7, dans laquelle la quantité de l'acide non polymérique (c) ayant au moins deux valeurs de pKa ou un ou plusieurs sels de celui-ci dans la composition est de 0,001 % à 10 % en poids, préférablement de 0,005 % à 5 % en poids, et plus préférablement de 0,01 % à 1 % en poids, par rapport au poids total de la composition.

9. Composition selon l'une quelconque des revendications 1 à 8, dans laquelle la quantité de l'eau (d) dans la composition est de 50 % à 99 % en poids, de préférence de 60 % à 98 % en poids, et plus préférentiellement de 70 % à 97 % en poids, par rapport au poids total de la composition.

10. Composition selon l'une quelconque des revendications 1 à 9, le pH de la composition allant de 3 à 9, préférablement de 3,5 à 8,5 et plus préférablement de 4 à 8.

11. Composition selon l'une quelconque des revendications 1 à 10, dans laquelle la composition est une composition cosmétique, préférablement une composition cosmétique cutanée, et plus préférablement une composition cosmétique de soins de la peau.

12. Procédé cosmétique pour une matière kératinique, telle que la peau, comprenant l'application sur la matière kératinique de la composition selon l'une quelconque des revendications 1 à 11 ; et
le séchage de la composition pour former un film cosmétique sur la substance kératinique.

13. Utilisation de (a) au moins un polymère cationique, le polymère cationique (a) étant choisi parmi les chitosanes ayant un poids moléculaire de 20 000 ou moins, dans une composition comprenant :
(b) au moins un polymère anionique ;
(c) au moins un acide non polymérique ayant au moins deux valeurs de pKa ou un ou plusieurs sels de celui-ci ; et
(d) de l'eau ;
afin de stabiliser la composition et/ou d'améliorer la facilité d'étalement de la composition.

14. Film, de préférence film cosmétique, comprenant :
(a) au moins un polymère cationique ;
(b) au moins un polymère anionique ; et
(c) au moins un acide non polymérique ayant au moins deux valeurs de pKa ou un ou plusieurs sels de celui-ci, dans lequel
le polymère cationique (a) est choisi parmi les chitosanes ayant un poids moléculaire de 20 000 ou moins.

15. Utilisation de (a) au moins un polymère cationique, le polymère cationique (a) étant choisi parmi les chitosanes ayant un poids moléculaire de 20 000 ou moins, dans un film comprenant :
(b) au moins un polymère anionique ; et
(c) au moins un acide non polymérique ayant au moins deux valeurs de pKa ou un ou plusieurs sels de celui-ci, afin d'améliorer la texture du film, de préférence afin d'améliorer la sensation d'hydratation, la douceur et le lissé apportés par le film et/ou pour réduire le caractère collant du film, et plus préférablement afin de réduire le caractère collant du film.
